# EUROPEAN PATENT SPECIFICATION

(11) **EP 0 474 607 B1**
(45) Date of publication and mention of the grant of the patent: **22.02.1995**
(21) Application number: 91830364.5
(22) Date of filing: 05.09.1991
(51) Int. Cl.: G01N 1/10, G01N 33/18, G01N 21/11, G01N 21/75

(54) **Apparatus for the discontinuous on-line field or process colorimetric chemical monitoring of liquid samples**
Vorrichtung zur diskontinuierlichen, kolorimetrischen, chemischen on-line Feld- oder Prozessüberwachung von flüssigen Proben
Appareil pour la surveillance discontinue en ligne, colorimétrique, chimique à champ ou processus, d'échantillons liquides

(30) Priority: 05.09.1990 IT 948290
(43) Date of publication of application: 11.03.1992
(73) Proprietor: Bedendo, Armando, I-45100 Rovigo (IT); Taschini, Roberto, I-50022 Greve in Chianti/Frazione Panzano (Firenze) (IT)
(72) Inventor: Bedendo, Armando, I-45100 Rovigo (IT); Taschini, Roberto, I-50022 Greve in Chianti/Frazione Panzano (Firenze) (IT)
(74) Representative: Mannucci, Gianfranco, Dott.-Ing.

(56) References cited:
- DE-A- 3 221 063
- US-A- 3 294 490
- ANALYTICAL CHEMISTRY vol. 59, no. 20, 15 October 1987, WASHINGTON,DC,USA pages 2534 - 2535; D.E.BAUTZ ET AL: 'Automated Sample Cell Cleaner'

## Description

The invention relates to a field or process analysing apparatus for performing colorimetric analyses using a discontinuous on-line system on liquid samples, essentially water - surface water, drinking water, waste water and sea water - for carrying out on site, that is "in the field", according to the preamble of claim 1.

An apparatus of this kind is known from DE-A-32 21 063. In this known apparatus the cell has an upper portion with a reduced cross section, in correapondence of which a discharge tube is arranged. The position of this tube defines the amount of sample which can be collected in the cell. Metering pipes are also provided for metering chemical reactants in the sample. Said pipes extend toward the bottom of the cell, in order that the minimum quantities of reactants metered therefrom are not lost through the discharge tube. After stirring of the sample and reactants, the mixture is made to flow out of the cell into a colorimetric measuring apparatus arranged under the cell. After measuring the sample is finally discharged from the apparatus.

This known apparatus requires long time for performing each measurement and moreover lacks reliability due to the relatively high number of parts.

The object of the invention is to provide instrumentation capable of satisfying the essential characteristics of field and process monitoring, which are:
- reliability;
- accuracy and precision;
- low servicing frequency with consequently low running costs;
- safety and speed of operation with a minimum number of compounds that are all simple and reliable.

These and other objects and advantages, which will be clear from the following text, are achieved by the combination of features of claim 1.

In the apparatus of claim 1 the means for lowering the level of the sample in the cell may advantageously comprise a fast stirrer arranged in the bottom of the cell and able to create in the cell a rising current causing the discharge over the spill rim of a constant amount of sample, depending on the speed at which said fast stirrer is run. Basically, the stirrer causes a forced overflowing of the liquid from the open cell by a centrifugal effect. How much sample remains in the cell is determined by the stirrer's top speed.

A single stirrer may be provided, running at high speed to create a centrifugal force that will cause the discharging over the spill rim of said constant fraction of the sample and the consequent lowering of the level in the cell, and running at reduced speed to stir the sample after the reagents have been added. The stirrer may comprise a rotor operated from the outside by a magnetic drive.

As an alternative, the means for lowering the level in the cell may comprise means for temporarily reducing the volume of the cell, by deformation of the walls and/or by immersion of a foreign body. In particular, it is possible to have a section of the cell made of an elastic and flexible material. Outside the cell is a pair of pincher jaws controlled by a stepping motor or the like.

The apparatus may comprise a feed pump, with a free discharge in parallel with the cell to create a hydraulic head to provide a flow into the cell from its bottom. This produces a constant pressure at the entrance to the cell.

Advantageously the spill rim may be of a continuous shape and surrounded by a discharge collector, with free discharge, optionally at two different levels to allow a wide margin of sample overspill during overflow.

The apparatus may comprise a number of cells, fed by the same pump and with an optional single free discharge, to create a hydraulic head for all the cells. Further characteristics are indicated in the appended claims.

The description and the attached drawings, which latter shows a practical non-limiting illustrative embodiment of the invention, will provide a clearer understanding of said invention. In the drawings:
Fig. 1 shows a general diagram;
Figs 2 and 3 show in detail a cell in vertical and horizontal section;
Fig. 4 shows a possible block diagram of an example of an analytical procedure; and
Figs 5A, 5B and 5C show the stages of producing a precise amount of the sample.

In a form illustrated in the drawing, 1 indicates a pump for taking up the liquid, for example water, for analysis, the pump 1 being able to run in continuous or intermittent mode, as appropriate for the material for analysis. The pump delivery tube 3 may feed at least one apparatus or alternatively several apparatuses simultaneously, and in either case a branch 3A of the tube 3 is provided for an overflow with a spill rim at the top in a cavity 5 from which excess liquid is discharged through the tube 7 and returned or thrown away. Obviously, the liquid for analysis is available in any amount, even greatly in excess of what is needed for the apparatus to examine. Between the pump 1 and the delivery tube 3 a filter 2 is inserted.

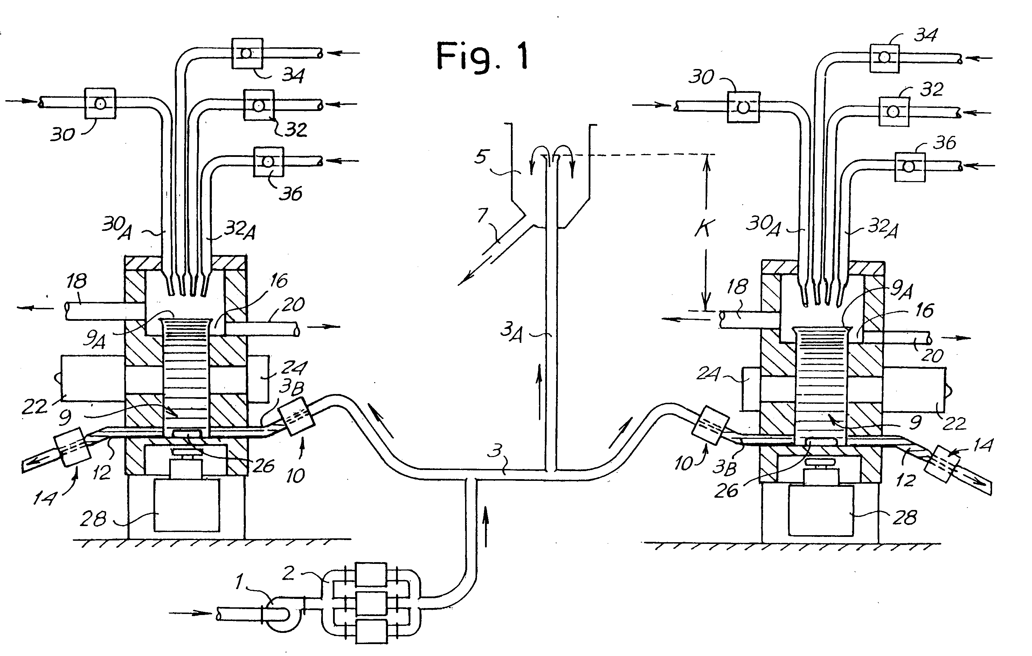

The tube 3 feeds a cell 9 that is arranged at a lower level than the free discharge of the tube 3A, so as to create in said cell a fixed head K; the tube 3 may also feed a number of cells, for example two cells shown in the diagram in Fig. 1. The cell may be of any size according to its use. The liquid is fed in by a tube 3B which introduces it into the cell 9 from the base, through a valve 10 which is advantageously a "pinch" type valve on a flexible elastic tube. A discharge 12 from the base is also provided for the cell 9 and it too is provided with a pinch valve 14 on the flexible tube. The cell 9 is open at the top and has a spill rim 9A surrounded by a sample collector 16 whence a discharge 18 at a higher level, and a discharge 20 at a lower level, lead off. The discharge 18 may be adequate for the excess liquid that discharges over the spill rim, while the discharge 20 carries off a fraction of the contents of the cell when completely full, the discharging of this fraction being brought about in the manner indicated below. At an intermediate position the cell has a system of chromatic examination by transparency, comprising on one side a light source 22 and on the other a photoelectric cell 24 which is part of an optoelectronic system capable of picking up light energy and converting it into electrical energy in order to generate signals from which it is possible to obtain the data for the analysis of each particular sample contained in the cell 9, with the addition of specific reagents to give the preselected chromatic reaction, in a manner known per se.

In the bottom of the cell 9, a rotor 26 is provided as a means of stirring the liquid contained in the cell. The rotor 26 is advantageously a magnetically driven rotor operated by a motor 28 outside the cell, that is with regard to the rotor 26, separate from the bottom of the cell of nonmagnetic material, so that there is no danger of leakages or contamination. The motor 28 is a two-speed motor for the purposes indicated below.

In the example illustrated, the apparatus also comprises two systems for feeding precise amounts of reagents, for example peristaltic pumps indicated by 30 and 32 to provide very precise amounts of the reagents that are to be added to the sample contained in the cell 9. Clearly, some other number of such systems may be provided. The reagents may for example be set up to analyse the orthophosphate parameter and hence one reagent may be ammonium molybdate in acidic solution, while a second reagent may be an organic reducing agent, for example ascorbic acid. A third feed with a peristaltic pump 34 may be provided to examine the control and calibration standard, using for example, for the above-mentioned parameter, potassium dihydrogen phosphate. A feed is also provided for the chemical washing of the cell, for example with a 50% aqueous-alcoholic solution and/or sodium hypochlorite or some other reagent, which may be performed with a high-capacity peristaltic pump as diagrammatically shown at 36.

The peristaltic pumps may be replaced by syringe means with a stepping motor and control valves.

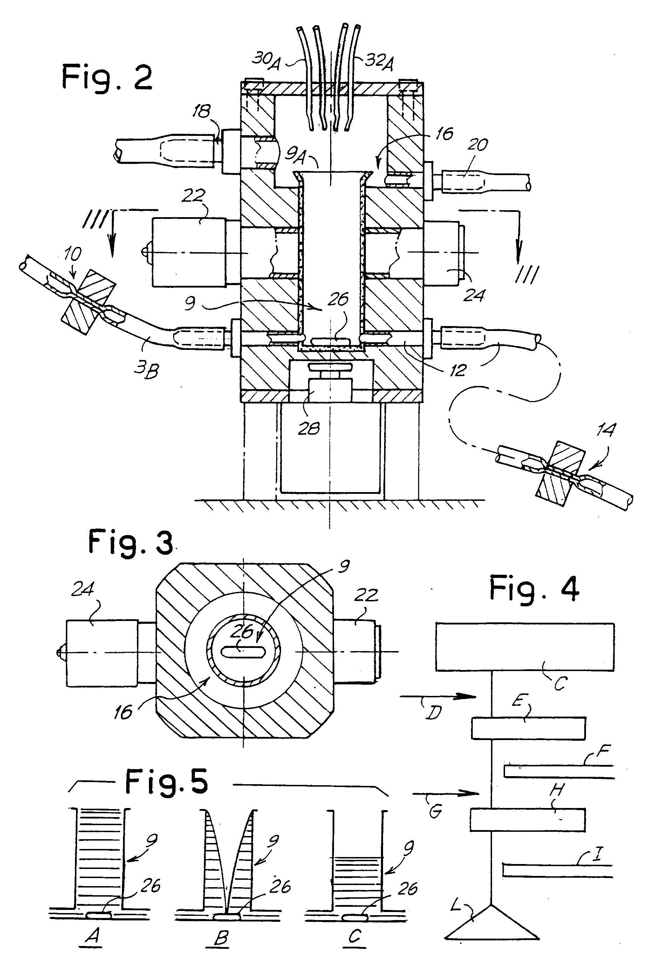

The sample is fed into the cell 9 - which is suitably chemically washed beforehand, as well as being washed with the same liquid that forms the sample - until filled up to the rim 9A, hence with a very precise amount, the excess being discharged from the collector 16 and/or 18 by natural overflow. Since the reagents have to be added into the cell to perform the analyses, the amount of sample in the cell 9 must be reduced to allow the abovementioned reagents to be added by the peristaltic pumps 30 and 32 successively. For this purpose, after the cell has been filled to the brim, the motor 28 is turned on at high speed, causing the rotor 26 to create a violent centrifugal movement of the sample liquid which is thrown over the rim 9A into the collector 16 to be removed, in an amount that is constant; when the fast action of the stirrer 26 ceases, the sample remaining in the cell has been reduced by that constant fraction that has been discharged by the fast action of the stirrer 26. Basically, therefore, a precise amount of the sample has been produced by forced or centrifugal overflow through the high-speed magnetic drive of the rotor 26. The centrifugal overflow due to the rising current discharges some of the sample from the cell in a way which is always constant and dependent exclusively on the top speed of the rotor 26. This principle of operation is diagram-matically illustrated in Figs 5A, 5B and 5C.

At this point, the reagents can be added successively by the peristaltic pumps 30 and then 32 and after each reagent has been added the mixture is stirred by the rotor 26 running at a slower speed to bring about stirring only and hence the first reaction stage, after which an optical reading of white is taken by the unit 22, 24, followed by addition of the second reagent, stirring and then the second reading as a sample dependent on the chromatic development.

The cycle requires the valve 14 to be closed, the valve 10 to be opened and then the cell to be filled to the brim with the sample, which may for example be a sample in which the orthophosphates PO₄ are to be examined. The sample C is treated (see Fig. 4) at D with the first reagent, which may be for example (NH₄)₂ MO₄, that is ammonium molybdate in acidic solution; this is followed at E by mixing and at F by the optical reading. Then a second reagent G, which may be ascorbic acid, is added, it is mixed in at H, and a reading is taken of the sample at I, after which it is discharged at L. After the discharge, but before repeating another analysis, the cell is washed, first with an aqueous-alcoholic solution or other chemical wash, for example by the pump 36, followed by discharging through the opening of the valve 14 and then further washed with the liquid of the sample arriving down the tube 3 through the open valve 10 and later discharged from 12 and 14. The cell is then filled by closing the valve 14, the excess is discharged over the spill rim 9A, and the operations already described are then carried out.

Clearly, the same apparatus may be used to analyse a number of chemical parameters by changing the interference filters as appropriate to give the specific wavelength of the analysis, and by using specific reagents for the preselected parameter. The cell, or each cell, may also be put to a test as a standard, for example with potassium dihydrogen phosphate added by the pump 34.

If the apparatus is to be used for different kinds of analyses, a different number of peristaltic pumps may be provided to feed in the various reagents in sequence, each pump supplying along its own tube such as 30A or 32A of the pumps 30 and 32, and this tube discharges the amount metered out by said pump through the delivery tubes such as 30A and 32A, discharging into the cell 9 by gravity.

The liquids discharged from the apparatus may be thrown away onto the ground or gathered in some suitable way in collecting vessels, except in the case of the excess of the sample liquid, which can in each case be thrown away or returned.

The apparatus for each cell or for a group of cells will be equipped with an electronic microprocessor system for data storage, cyclical carrying out of the various stages of an operation, controlling the peristaltic pumps, and for all the other functions which are to be carried out by the apparatus itself and which must be autonomous and automatic, even as regards the remote transmission of stored and suitably processed data. It would be possible for a single electronic computer to run two or more apparatuses in parallel. In this case there may be just one set of controls to run the parts (valves, pumps, rotors) of both apparatuses.

A stage of standardising the apparatus may be programmed each time this is felt to be appropriate, or done automatically at a programmed frequency. This stage is carried out by means of the pump 34, as a peristaltic pump, which charges the cell from above up to the maximum level, to then follow the same procedure already described for the sample examination cycle. The chemical washing stage is performed by means of the pump 36, which may be a peristaltic pump, which feeds to the cell specific washing liquids according to the chemical nature of the reagents used in the analysis. After the chemical wash comes a direct wash with the same liquid from which the sample is then taken, for example on starting a new stage of analysis. To this end, while the cell 9 is being filled at the start of an analysis, it is possible for the valve 10 to stay open for a certain length of time with the rotor 26 on, while the liquid that has been fed in - after filling the cell - overflows from the rim 9B.

It will be understood that the drawing shows only an illustrative embodiment given solely by way of a practical demonstration of the invention, it being possible for said invention to vary as regards shapes and arrangements without thereby departing from the scope of the concept which underlies said invention. For example, the standard and the liquid for the chemical wash may be delivered by syphon rather than by a peristaltic pump.

## Claims

1. Transportable apparatus for field or process analyses of water or other liquid samples, particularly colorimetric analyses on a sample taken periodically and delivered to a measuring cell, and for the monitoring, and if required the remote transmission, of the data, including a cell (9) for the sample; feed means (3B, 10) for feeding the sample in to the cell; means (26, 28) for producing a precise amount of sample in the cell; metering means (30, 30A, 32, 32A, 34) for metering precise amounts of reactants into the cell; stirring means (26) for stirring the sample in said cell (9); measuring means (22, 24) for colorimetric analysis of the sample synchronized with said metering means and said stirring means; discharging means (12, 14) to discharge the sample from said cell base; washing means (30, 10) for washing said cell; characterised in that: said cell has an upper spill rim (9A); that said means (26, 28) for producing a precise amount of sample in the cell (9) include means (26, 28) to lower the level of the sample in the cell (9) by discharging over the spill rim (9A) a constant amount of sample; and that said measuring means (22, 24) perform the measuring on the sample inside the cell (9).

2. Apparatus according to claim 1, characterised in that said feed means (3B, 10) also form part of said washing means (30, 10), the washing taking place by discharging over the spill rim (9A) of the cell (9) and by the discharging means (12, 14), and washing products feeding means (36) also being provided for feeding washing products into the cell.

3. Apparatus according to claim 1 or 2, characterised in that said feed means (3B; 10) opens into the base of the cell.

4. Apparatus according to one or more of the preceding claims, characterised in that the means (26, 28) to lower the level of the sample in the cell comprise a fast stirrer (26) arranged in the bottom of the cell (9) and able to create in the cell a rising current causing a forced overflowing by a centrifugal effect with discharge over the spill rim (9A) of a constant amount of sample, depending on the speed at which said fast stirrer (26) is run.

5. Apparatus according to claim 4, characterised in that said stirring means (26, 28) include a stirrer (26) which can be rotated at two different rotational speeds, namely at a first high speed for the discharge of the sample by centrifugal overflow over said spill rim with consequent lowering of the level in the cell, and at reduced speed to stir the sample after the reagents have been added.

6. Apparatus according to either of claims 4 and 5, characterised in that the stirrer comprises a rotor (26) operated from the outside by a magnetic drive.

7. Apparatus according to claim 1, 2 or 3, characterised in that the means to lower the level of sample in the cell comprise means for temporarily reducing the volume of the cell, by deformation of the walls and/or by immersion of a foreign body into the cell.

8. Apparatus according to one or more of the preceding claims, characterised in that it comprises a feed pump (1), which is connected by a feeding tube (3) to said feed means (3B, 10) for feeding the sample in the cell(9); and that said feeding tube (3) has a branch (3A) with an open end forming an overflow rim, said overflow rim being higher than the spill rim (9A) of said cell to create a hydraulic head for the sample to flow into the cell from its bottom.

9. Apparatus according to one or more of the preceding claims, characterised in that the spill rim (9A) of the cell (9) is continuous in shape and is surrounded by a sample collector (16), in which the sample overflowing from the spill rim (9A) of the cell (9) is collected, said collector being provided with a discharge duct (18, 20), at one or two different levels.

10. Apparatus according to one or more of the preceding claims, characterised in that it comprises a plurality of cells (9), fed by the same pump (1) and with an optional single branch (3A) extending from the feeding tube of the pump, to create a hydraulic head for all the cells.

## Patentansprüche

1. Vorrichtung zur Feld- oder Prozeßanalyse von Wasser oder anderen flüssigen Proben, insbesondere zur kolorimetrischen Analyse einer periodisch entnommenen und einer Meßzelle zugeführten Probe zur Überwachung und falls erforderlich zur Fernübertragung der Daten, mit einer Zelle (9) für die Probe; Fördermittel (3B, 10) zur Zuführung der Proben in eine Zelle; Mittel (26, 28) zur Herstellung einer genauen Menge der Probe in der Zelle; Meßeinrichtungen (30, 30A, 32, 32A, 34) zur Abmessung genauer Mengen an Reaktionsmittel in der Zelle; Rühreinrichtungen (26) zum Umrühren der Probe in der Zelle (9); Meßeinrichtungen (22, 24) zur kolorimetrischen Analyse der Probe, die mit der Mengenmeßeinrichtung und der Rühreinrichtung synchronisiert ist, Entladeeinrichtungen (12, 14) zur Entladung der Probe vom Zellenboden aus; Wascheinrichtungen (30, 10) zum Waschen der Zelle; **dadurch gekennzeichent, daß** die Zelle einen oberen Überlaufrand (9A) aufweist; daß die Mittel (26, 28) zur Herstellung einer genauen Menge der Probe in der Zelle (9) Einrichtungen (26, 28) zur Absenkung des Niveaus der Probe in der Zelle (9) durch Entladung einer konstanten Probenmenge über den Überlaufrand (9A) aufweisen; und daß die Meßeinrichtungen (22, 24) die Messung an der Probe innerhalb der Zelle (9) ausführen.

2. Vorrichtung nach Anspruch 1, **dadurch gekennzeichnet, daß** die Fördermittel (3B, 10) auch einen Teil der Wascheinrichtungen (30, 10) bilden, wobei der Waschvorgang durch Entladung über den Überlaufrand (9A) der Zelle (9) und durch die Entladevorrichtung (12, 14) erfolgt, und daß die Zuführeinrichtungen (36) für die Waschprodukte auch für die Zuführung der Waschprodukte in die Zelle dienen.

3. Vorrichtung nach Anspruch 1 oder 2, **dadurch gekennzeichnet, daß** die Fördermittel (3B; 10) zum Boden der Zelle offen sind.

4. Vorrichtung nach einem oder mehreren der voranstehenden Ansprüche, **dadurch gekennzeichnet, daß** die Mittel (26, 28) zur Absenkung des Niveaus der Probe in der Zelle einen Schnellrührer (26) aufweisen, der im Boden der Zelle (9) angeordnet ist und welcher in der Lage ist in der Zelle einen ansteigenden Strom zu erzeugen, der durch die Zentrifugalwirkung einen zwangsweisen Überlauf bewirkt, so daß eine konstante Probenmenge über den Überlaufrand (9A) sich in Abhängigkeit von der Geschwindigkeit, mit der sich der Schnellrührer dreht, entlädt.

5. Vorrichtung nach Anspruch 4, **dadurch gekennzeichnet, daß** die Rührvorrichtung (26, 28) einen Rührer (26) aufweist, der mit zwei verschiedenen Drehgeschwindigkeiten betrieben werden kann, nämlich mit einer hohen Geschwindigkeit zur Entladung der Probe durch einen zentrifugalen Überlauf über den Überlaufrand mit der Folge einer Absenkung des Niveaus in der Zelle, und mit verringerter Geschwindigkeit zum Umrühren der Probe nach Zugabe der Reaktionsmittel.

6. Vorrichtung nach einem der Ansprüche 4 und 5, **dadurch gekennzeichnet, daß** der Rührer einen Rotor (26) aufweist, der durch einen magnetischen Antrieb von außen betätigt wird.

7. Vorrichtung nach Anspruch 1, 2 oder 3, **dadurch gekennzeichnet, daß** die Einrichtung zur Senkung des Niveaus der Probe in der Zelle Mittel zur kurzzeitigen Verringerung des Volumens der Zelle durch Deformation der Wände und/oder durch Eintauchen eines Fremdkörpers in die Zelle aufweist.

8. Vorrichtung nach einem oder mehreren der vorstehenden Ansprüche, **dadurch gekennzeichnet, daß** sie eine Zuführpumpe (1) aufweist, die über ein Zuführrohr (3) mit den Fördermitteln (3B, 10) zur Zuführung der Probe in die Zelle (9) verbunden ist; und daß das Zuführrohr (3) einen Abzweig (3A) mit einem offenen Ende aufweist, das einen Überlaufrand bildet, wobei der Überlaufrand höher liegt als der Überlaufrand (9A) der Zelle, um eine hydraulische Fallhöhe zu erzeugen, so daß die Probe vom Boden in die Zelle fließen kann.

9. Vorrichtung nach einem oder mehreren der voranstehenden Ansprüche, **dadurch gekennzeichnet, daß** der Überlaufrand (9A) der Zelle (9) gleichförmig ausgebildet ist und von einem Probensammler (16) umgeben ist, in dem die über den Überlaufrand (9A) fließende Probe der Zelle (9) gesammelt wird, wobei der Sammler mit Entladeleitungen in einer Höhe oder zwei unterschiedlichen Höhen versehen ist.

10. Vorrichtung nach einem oder mehreren der voranstehenden Ansprüche, **dadurch gekennzeichnet, daß** sie mehrere Zellen (9) aufweist, die von der gleichen Pumpe (1) versorgt werden und mit einem wahlweisen einzigen Zweig (3A), der sich von dem Zuführrohr der Pumpe erstreckt, um für alle Zellen eine hydraulische Fallhöhe zur Verfügung zu stellen.

## Revendications

1. Dispositif transportable pour des analyses sur le terrain ou en processus d'échantillons d'eau ou autres liquides, en particulier des analyses colorimétriques sur un échantillon prélevé périodiquement et introduit dans une cuve de mesure, et pour le contrôle, et si nécessaire la télétransmission, des données, comprenant une cuve (9) pour l'échantillon; des moyens d'acheminement (3B, 10) pour acheminer l'échantillon jusque dans la cuve; des moyens (26, 28) pour produire une quantité précise d'échantillon dans la cuve; des moyens de dosage (30, 30A, 32, 32A, 34) pour doser des quantités précises de réactifs introduits dans la cuve; un moyen d'agitation (26) pour agiter l'échantillon dans ladite cuve (9); des moyens de mesure (22, 24) pour l'analyse colorimétrique de l'échantillon en synchronisme avec lesdits moyens de dosage et ledit moyen d'agitation; des moyens d'évacuation (12, 14) pour évacuer l'échantillon depuis la base de ladite cuve; des moyens de lavage (30, 10) pour laver ladite cuve; caractérisé en ce que ladite cuve a un bord supérieur de déversement (9A), en ce que lesdits moyens (26, 28) pour produire une quantité précise d'échantillon dans la cuve (9) comprennent des moyens (26, 28) pour abaisser le niveau de l'échantillon dans la cuve (9) en évacuant par dessus le bord (9A) de déversement une quantité constante d'échantillon, et en ce que lesdits moyens de mesure (22, 24) effectuent les mesures sur l'échantillon à l'intérieur de la cuve (9).

2. Dispositif selon la revendication 1, caractérisé en ce que lesdits moyens d'acheminement (3B, 10) font aussi partie desdits moyens de lavage (30, 10), le lavage s'effectuant par évacuation par dessus le bord de déversement (9A) de la cuve (9) et par les moyens d'évacuation (12, 14), un moyen d'alimentation (36) en produits de lavage étant également prévu pour acheminer des produits de lavage jusque dans la cuve.

3. Dispositif selon la revendication 1 ou 2, caractérisé en ce que lesdits moyens d'acheminement (3B; 10) débouchent dans la base de la cuve.

4. Dispositif selon une ou plusieurs des revendications précédentes, caractérisé en ce que les moyens (26, 28) pour abaisser le niveau de l'échantillon dans la cuve comprennent un agitateur rapide (26) disposé au fond de la cuve (9) et apte à créer dans la cuve un courant ascendant provoquant un débordement forcé par un effet centrifuge avec évacuation d'une quantité constante d'échantillon, par dessus le bord de déversement (9A), selon la vitesse à laquelle fonctionne ledit agitateur rapide (26).

5. Dispositif selon la revendication 4, caractérisé en ce que lesdits moyens d'agitation (26, 28) comprennent un agitateur (26) qui peut tourner à deux vitesses de rotation différentes, à savoir à une première vitesse élevée pour l'évacuation de l'échantillon par débordement centrifuge par dessus ledit bord de déversement avec une baisse consécutive du niveau dans la cuve, et à vitesse réduite pour agiter l'échantillon après que les réactifs ont été ajoutés.

6. Dispositif selon l'une ou l'autre des revendications 4 et 5, caractérisé en ce que l'agitateur comporte un rotor (26) actionné de l'extérieur par un moyen d'entraînement magnétique.

7. Dispositif selon la revendication 1, 2 ou 3, caractérisé en ce que les moyens pour abaisser le niveau d'échantillon dans la cuve comportent un moyen pour réduire temporairement le volume de la cuve, par déformation des parois et/ou par immersion d'un corps étranger dans la cuve.

8. Dispositif selon une ou plusieurs des revendications précédentes, caractérisé en ce qu'il comprend une pompe d'alimentation (1), qui est reliée auxdits moyens d'acheminement (3B, 10) par un tuyau d'alimentation (3) pour acheminer l'échantillon dans la cuve (9), et en ce que ledit tuyau d'alimentation (3) a une ramification (3A) avec une extrémité ouverte formant un bord de débordement, ledit bord de débordement étant plus haut que le bord de déversement (9A) de ladite cuve afin de créer une pression de colonne d'eau pour que l'échantillon pénètre dans la cuve par le bas de celle-ci.

9. Dispositif selon une ou plusieurs des revendications précédentes, caractérisé en ce que le bord de déversement (9A) de la cuve a une forme continue et est entouré par un collecteur (16) d'échantillon, dans lequel l'échantillon débordant par dessus le bord de déversement (9A) de la cuve (9) est recueilli, ledit collecteur comportant un conduit d'évacuation (18, 20), à un ou deux niveaux différents.

10. Dispositif selon une ou plusieurs des revendications précédentes, caractérisé en ce qu'il comprend plusieurs cuves (9), alimentées par la même pompe (1), une ramification unique facultative (3A) s'étendant depuis le tuyau d'alimentation de la pompe, afin de créer une pression de colonne d'eau pour toutes les cuves.
